(19) Europäisches Patentamt

European Patent Office

Office européen des brevets

(11) Veröffentlichungsnummer : **0 327 007 B1**

(12) # EUROPÄISCHE PATENTSCHRIFT

(45) Veröffentlichungstag der Patentschrift :
**09.09.92 Patentblatt 92/37**

(21) Anmeldenummer : **89101596.8**

(22) Anmeldetag : **31.01.89**

(51) Int. Cl.⁵ : **C07C 303/24, C07C 303/44, C07C 305/04, C07C 305/06**

(54) **Verfahren zur Herstellung sulfatierter Alkanol- oder Alkylphenol-Oxethylate mit erniedrigtem Gehalt an 1,4-Dioxan.**

(30) Priorität : **03.02.88 DE 3803110**

(43) Veröffentlichungstag der Anmeldung :
**09.08.89 Patentblatt 89/32**

(45) Bekanntmachung des Hinweises auf die Patenterteilung :
**09.09.92 Patentblatt 92/37**

(84) Benannte Vertragsstaaten :
**AT BE CH DE ES FR GB GR IT LI NL SE**

(56) Entgegenhaltungen :
**EP-A- 0 052 801**
**DD-A- 135 381**
**DE-A- 3 126 175**
**PATENT ABSTRACTS OF JAPAN, Band 6, Nr. 15 (C-89)(893), 28. Januar 1982 & JP-A-56 138 164 & JP-A-65 138 163**

(73) Patentinhaber : **HOECHST AKTIENGESELLSCHAFT**
**Postfach 80 03 20**
**W-6230 Frankfurt am Main 80 (DE)**

(72) Erfinder : **Aigner, Rudolf**
**Hochkalterstrasse 7**
**W-8269 Burgkirchen (DE)**
Erfinder : **Müller, Günther, Dr.**
**Kampenwandstrasse 13**
**W-8269 Burgkirchen (DE)**
Erfinder : **Müller, Rainer**
**Robert-Koch-Strasse 86**
**W-8263 Burghausen (DE)**
Erfinder : **Reuner, Horst**
**Moosbrunnerstrasse 18**
**W-8263 Burghausen (DE)**

## Beschreibung

Die Erfindung betrifft ein Verfahren zur Herstellung sulfatierter Alkanol- oder Alkylphenol-Oxethylate mit erniedrigtem Gehalt an 1,4-Dioxan nach Anspruch 1.

Sulfatierte Alkanol- oder Alkylphenol-Oxethylate werden technisch hergestellt durch Umsetzung von Alkanolen oder Alkylphenolen mit Ethylenoxid und nachfolgende Sulfatierung der Anlagerungsprodukte mit Mitteln wie Chlorsulfonsäure oder Schwefeltrioxid. Hierbei entstehen Schwefelsäurehalbester, die in der Regel unmittelbar anschließend mit alkalischen Verbindungen, beispielsweise Alkalihydroxiden, in wäßriger Lösung neutralisiert werden. Die so hergestellten, mit einem Sammelnamen als "Ethersulfate" bezeichneten Verbindungen sind wertvolle Tenside für die verschiedensten Anwendungsgebiete, sie werden beispielsweise auch als Körperpflegemittel verwendet. Bei der Sulfatierung der Alkanol- oder Alkylphenol-Oxethylate entsteht unter anderem als Nebenprodukt 1,4-Dioxan. Wird bei der technisch häufig angewendeten Sulfatierung mit Schwefeltrioxid ein Reaktor älterer Bauart benützt, wie es noch vielfach der Fall ist, so enthalten die sulfatierten Oxethylate in Abhängigkeit von ihrem Oxethylatgehalt etwa 0,2 bis 0,03Gew.-% 1,4-Dioxan. Moderne Reaktoren ermöglichen zwar niedrigere 1,4-Dioxangehalte, etwa in der Größenordnung von 0,07 bis 0,005 Gew.-%, doch reicht dies für bestimmte Anwendungen für die Erfordernisse des Marktes nicht aus.

Aus toxikologischen Untersuchungen von R. J. Kociba et al., Toxicology, Applied Pharmacology, Vol. 30 (1974), Seiten 275 bis 298, geht hervor, daß 1,4-Dioxan im Tierversuch, allerdings erst bei Anwendung sehr großer Mengen, gesundheitliche Schädigungen erzeugt. Obwohl aus diesen Versuchen keine direkte Gefahr bei der Verarbeitung und Anwendung von Ethersulfaten herleitbar ist, besteht doch seither bei Herstellern und Anwendern der Ethersulfate, vor allem bei der Anwendung im kosmetischen Bereich, ein großes Interesse an Produkten, die einen möglichst weitgehend verminderten Gehalt an 1,4-Dioxan aufweisen, um jedes Risiko auszuschalten. Es besteht daher die Aufgabe, den Gehalt an 1,4-Dioxan in Ethersulfaten, wenn möglich schon in deren Vorprodukten, möglichst kostengünstig zu erniedrigen.

Zur Herabsetzung des 1,4-Dioxan-Gehaltes in Ethersulfaten sind verschiedene Verfahren bekannt, die alle eine Entfernung es 1,4-Dioxans durch Wärme beziehungsweise Wasserdampfbehandlung des fertigen Ethersulfates bezwecken. So wird in der US-PS 4,285,881 ein Verfahren beschrieben, Ethersulfat/Dioxan-Mischungen mit dioxanfreiem Wasserdampf, bei Temperaturen von 25 bis 150 °C, in einer Abstreif-Vorrichtung in Kontakt zu bringen, wobei die Ethersulfat/Dioxan-Mischung vorteilhaft in dünner Schicht fließend angewendet wird. Nach DE 31 26 175-A1 werden Ethersulfate in konzentrierter 60 bis 80 gew.%iger wäßriger Lösung einer Wärmebehandlung bei 50 bis 130 °C unter vermindertem Druck unterworfen, um sie geruchfrei zu machen, wobei auch bei der Sulfatierung als Nebenprodukte gebildete cyclische Ether (1,4-Dioxan) entfernt werden. In DE 30 44 488-A1 ist ein ähnliches Verfahren beschrieben, mit dem direkt genannten Ziel, Ethersulfate mit erniedrigtem Gehalt an 1,4-Dioxan zu erzeugen. Diese Verfahren sind apparativ aufwendig und benötigen nicht unbeträchtliche Energiemengen. Dieser Aufwand könnte erheblich vermindert werden, wenn der 1,4-Dioxangehalt des sulfatierten Produktes schon vor der Neutralisation deutlich niedriger wäre als bisher üblich.

Es wurde nun einer Verfahren gefunden, das ohne größeren apparativen Mehraufwand in den vorhandenen Vorrichtungen zur Sulfatierung mit Schwefeltrioxid durchgeführt werden kann, wobei sulfatierte Produkte mit deutlich herabgesetztem Gehalt an 1,4-Dioxan entstehen, so daß ein nachträglicher Reinigungsaufwand entweder entfällt oder bedeutend vermindert werden kann.

Das neue Verfahren zur Herstellung sulfatierter Alkanol- oder Alkylphenol-Oxethylate, wobei mindestens ein flüssiges Alkanol-Oxethylat, das 6 bis 22 C-Atome im Alkanolrest enthält, oder mindestens ein Alkylphenol-Oxethylat, das 1 bis 3 Alkylgruppen und insgesamt 8 bis 18 C-Atome in den Alkylgruppen enthält, mit einem Gasgemisch, das neben mindestens einem Inertgas 1 bis 8 Vol.-%, bezogen auf das Gasgemisch, gasförmiges $SO_3$ enthält, im Verhältnis von 0,9 bis 1 mol $SO_3$ je 1 mol OH-Gruppen im Alkanol- oder Alkylphenol-Oxethylat unter Kühlung in Kontakt gebracht wird, nach Beendigung der Reaktion das flüssige Reaktionsgemisch und das Gas voneinander getrennt werden und das flüssige Reaktionsgemisch mit wäßrigem Alkalihydroxid, Magnesiumhydroxid, Ammoniumhydroxid oder substituiertem Ammoniumhydroxid neutralisiert wird, dadurch gekennzeichnet, daß dem Reaktionsgemisch nach Beendigung der Reaktion mit $SO_3$, jedoch vor der Trennung von flüssigem Reaktionsgemisch und Gas 0,1 bis 5 Gew.-%, bezogen auf eingesetztes Alkanol- oder Alkylphenol-Oxethylat, von mindestens einer der folgenden Verbindungen: Wasser, Ethanol, Propanol-1, Propanol-2, n-Heptan zugemischt werden, die mittlere Verweilzeit des Reaktionsgemisches von der Zugabe mindestens einer der genannten Verbindungen bis zur Abführung des flüssigen Reaktionsgemisches zur Neutralisation 60 Minuten nicht überschreitet und die Temperatur bei der Trennung von Flüssigkeit und Gas 20 bis 60 °C beträgt.

Für das erfindungsgemäße Verfahren sind Alkanol-Oxethylate geeignet, die 6 bis 22 C-Atome im Alkanolrest enthalten. Vorzugsweise werden solche Alkanol-Oxethylate eingesetzt, deren Alkanolrest 8 bis 18 und insbesondere 10 bis 16 C-Atome aufweisen. Geeignete Alkylphenol-Oxethylate haben 1 bis 3 Alkylgruppen am Phenolrest gebunden, die ihrerseits insgesamt 8 bis 18 C-Atome aufweisen.

EP 0 327 007 B1

Die Zahl der -CH$_2$CH$_2$O-Gruppen in den Alkanol- oder Alkylphenol-Oxethylaten kann in weiten Grenzen schwanken, es können beispielsweise 1 bis 15 solcher Gruppen an einen Alkanol- oder Alkylphenolrest gebunden sein. Bevorzugt werden solche Verbindungen eingesetzt, bei denen 1 bis 6 und insbesondere solche, bei denen 2 bis 4 -CH$_2$CH$_2$O-Gruppen im Molekül vorliegen.

Die Oxethylate werden mit einem Gasgemisch, das neben mindestens einem Inertgas 1 bis 8 Vol.-%, bezogen auf das Gasgemisch, gasförmiges SO$_3$ enthält, umgesetzt, wobei je 1 mol OH-Gruppen im Alkanol- oder Alkylphenol-Oxethylat 0,9 bis 1 mol SO$_3$ angewendet werden. Da häufig Gemische verschiedener Alkanol- oder Alkylphenol-Oxethylate mit SO$_3$ umgesetzt werden, wird die anzuwendende Menge SO$_3$ zweckmäßig aufgrund der OH-Zahl des Oxethylatgemisches ermittelt. Prinzipiell ist es möglich, Gasmischungen mit weniger als 1 Vol.-% SO$_3$ zu verwenden, jedoch verschlechtert sich dann die Raum-Zeit-Ausbeute unnötig. Inertgasmischungen mit mehr als 8 Vol.-% SO$_3$ führen im allgemeinen zu Schwierigkeiten durch ungleichmäßige Sulfatierung, mangelnde Temperaturkonstanz und steigende Bildung von unerwünschten Nebenprodukten. Vorzugsweise wird ein Gasgemisch verwendet, das 1,5 bis 5 Vol.-%, bezogen auf das Gasgemisch, gasförmiges SO$_3$ enthält.

Obwohl auch andere Inertgase geeignet sind, werden in der Regel wegen der leichten Beschaffbarkeit Luft oder Stickstoff bevorzugt. Die Umsetzung des flüssigen Ethoxylates oder Ethoxylatgemisches mit dem SO$_3$ enthaltenden Inertgas, wird meist in sogenannten Fallfilmreaktoren durchgeführt, in denen ein an einer gekühlten Wandung in dünner Schicht herabrieselnder Flüssigkeitsfilm mit den Gas im Gleichstrom in Kontakt gebracht wird. Als weitere mögliche Reaktoren wären beispielsweise Kesselkaskaden geeignet. Das bei der Umsetzung entstehende Flüssigkeits-Gasgemisch wird nach Beendigung der Reaktion in der Regel in einem Zentrifugalabscheider (Zyklon) in flüssiges Reaktionsgemisch und Gas, das nur noch Spuren SO$_3$ enthält, getrennt.

Das flüssige Reaktionsgemisch wird unmittelbar anschließend mit wäßrigem Alkali-, Magnesium- oder gegebenenfalls substituiertem Ammoniumhydroxid neutralisiert. Je nach der hierbei eingesetzten Wassermenge entstehen so wasserhaltige Konzentrate mit einem Ethersulfatgehalt von bis zu 80 Gew.-%, bezogen auf das Konzentrat, oder konzentrierte wäßrige Lösungen mit Ethersulfatgehalten von etwa 15 bis etwa 35 Gew.-%, bezogen auf die konzentrierte Lösung. Geringere Konzentrationen sind zwar ohne weiteres herstellbar, aber unvorteilhaft wegen des vergleichsweise hohen Transportvolumens.

Neben unsubstituiertem Ammoniumhydroxid kann auch substituiertes zur Neutralisation verwendet werden, beispielsweise ein Ammoniumhydroxid, das mit 1 bis 4 Alkylgruppen substituiert ist, die ihrerseits jede für sich 1 bis 4 Kohlenstoff-Atome enthalten können, auch mit Benzyl- oder Hydroxyalkylgruppen substituierte Ammoniumhydroxide sind geeignet. Letztere enthalten in der Hydroxyalkylgruppe zweckmäßig 2 bis 4, vorzugsweise 2 C-Atome.

Erfindungsgemäß werden dem Reaktionsgemisch, bestehend aus Flüssigkeit und Gas, das nach der Beendigung der Reaktion mit SO$_3$ anfällt, jedoch vor der Trennung dieses Gemisches in Gas und Flüssigkeit 0,1 bis 5 Gew.-%, bezogen auf eingesetztes Alkanol- oder Alkylphenol-Oxethylat, von mindestens einer der folgenden Verbindungen: Wasser, Ethanol, Propanol-1, Propanol-2 oder n-Heptan zugemischt und die Temperatur so eingestellt, daß sie bei der Trennung von Flüssigkeit und Gas 20 bis 60 °C beträgt. Wegen der leichten Beschaffbarkeit und günstigen Wirkung wird bevorzugt Wasser verwendet.

Die Temperatur, bei der die Trennung von Gas und Flüssigkeit erfolgt, wird zweckmäßig bereits bei der Umsetzung von SO$_3$ mit dem Alkanol- oder Alkylphenol-Oxethylat eingestellt, es kann jedoch auch bei dieser Umsetzung eine niedrigere Temperatur gefahren werden, um die Bildung von Nebenprodukten zu vermindern, und die Mischung mit den zuvor genannten Zusatzstoffen, beispielsweise Wasser, erst kurz vor der Trennung von Gas und Flüssigkeit auf die gewünschte Temperatur gebracht werden. Unterhalb 20 °C ist der Effekt der Abtrennung des 1,4-Dioxans im allgemeinen zu gering, über 60 °C treten unerwünschte Nebenreaktionen, beispielsweise die Bildung zusätzlichen 1,4-Dioxans ein. Vorzugsweise findet die Trennung von Gas und Flüssigkeit bei einer Temperatur von 25 bis 45 °C statt.

Gute Ergebnisse werden erhalten, wenn 0,2 bis 2 Gew.-%, bezogen auf eingesetztes Alkanol- oder Alkylphenol-Oxethylat, von mindestens einer der Verbindungen: Wasser, Ethanol, Propanol-1, Propanol-2 oder n-Heptan (nachfolgend der kürze halber mit "Zusatzstoffe" bezeichnet) der Reaktionsmischung nach der Reaktion mit SO$_3$ zugesetzt werden. Es können auch Mischungen von zwei oder mehreren Zusatzstoffen verwendet werden.

Die Eingabe des Zusatzstoffs oder der Zusatzstoffe in die meist als Schaum mit beträchtlicher Strömungsgeschwindigkeit ankommende Reaktionsmischung erfolgt zweckmäßig schnell unter guter Durchmischung, beispielsweise indem durch Verengung des Durchtrittsquerschnitts die Strömungsgeschwindigkeit der Zusatzstoffe erhöht wird. Geeignet sind insbesondere Düsen, die den Zusatzstoff in feine Tröpfchen verteilen. Die Einströmungsrichtung des Zusatzstoffes wird vorteilhaft so gewählt, daß in der Reaktionsmischung Turbulenzen entstehen, auch kann sich die Ausbildung einer kreisförmigen Strömung senkrecht zur Strömungsrichtung der Reaktionsmischung günstig auswirken, gegebenenfalls können statische Mischer oder Rührer

3

Verwendung finden.

Neben dem Zusatzstoff beziehungsweise den Zusatzstoffen können je 1 Vol.-Teil des eingesetzten, $SO_3$ enthaltenden Gasgemisches 0,5 bis 3 Vol.-Teile von mindestens einem Inertgas dem Reaktionsgemisch nach der Beendigung der Reaktion mit $SO_3$, jedoch vor der Trennung von flüssigem Reaktionsgemisch und Gas, zugegeben werden. Geeignete Inertgase sind beispielsweise Luft oder Stickstoff. Die Abscheidung des 1,4-Dioxans wird hierdurch vielfach noch verbessert. Die Zugabe des Inertgases erfolgt zweckmäßig ähnlich wie die Zugabe des Zusatzstoffes.

In einer bevorzugten Ausführungsform des erfindungsgemäßen Verfahrens werden auf 1 Gew.-Teil pro Stunde eingesetztes Alkanol- oder Alkylphenol-Oxethylat 0,5 bis 100 Gew.-Teile pro Stunde, vorzugsweise 5 bis 50 Gew.-Teile pro Stunde, des nach der Umsetzung mit $SO_3$ und Abtrennung des Gases erhaltenen flüssigen Reaktionsgemisches gekühlt, ein oder mehrere der weiter oben genannten Zusatzstoffe in den ebenfalls weiter oben angegebenen Mengen, bezogen auf eingesetztes Alkanol- beziehungsweise Alkylphenol-Oxethylat, zugegeben, zurückgeführt und dem Reaktionsgemisch, bestehend aus Flüssigkeit und Gas, nach der Beendigung der Umsetzung mit $SO_3$ wieder beigemischt.

Es ist zu berücksichtigen, daß bei der Abscheidung des Gases von dem flüssigen Reaktionsgemisch nicht aller Zusatzstoff gasförmig zusammen mit 1,4-Dioxan abgeführt wird, sondern sich zwischen abgeführtem Zusatzstoff und in der Reaktionsmischung verbliebenem Zusatzstoff ein Gleichgewicht einstellt, das unter anderem von der Temperatur und dem Druck während der Abscheidung abhängig ist. Die im flüssigen Reaktionsgemisch verbleibende Menge des Zusatzstoffs wird zweckmäßig nach Einstellung des Gleichgewichts bestimmt und, wenn es sich um Wasser handelt, bei der Wassermenge, die bei der Neutralisation zugesetzt wird, berücksichtigt. Der Zusatzstoff kann der gekühlten rückgeführten Flüssigkeit auf verschiedene Weise zugemischt werden, beispielsweise durch Einströmenlassen und anschließendes Führen über einen statischen Mischer, durch Einrühren oder durch Eindüsen.

Wenn nach dem Anfahren der Reaktion mit $SO_3$ die Menge der erzeugten Reaktionsmischung nach der Abscheidung des Gases die zur Rückführung der gekühlten Flüssigkeit benötigte Menge zu übersteigen beginnt, wird mit der Abführung eines Teils des flüssigen Reaktionsgemisches und dessen Neutralisation mit den weiter oben beschriebenen wäßrigen Alkalien begonnen.

Die mittlere Verweilzeit von der Zugabe des Zusatzstoffes beziehungsweise der Zusatzstoffe zum Reaktionsgemisch bis zur Abführung des flüssigen Reaktionsgemisches zur Neutralisation sollte 60 Minuten nicht übersteigen, da das noch nicht neutralisierte Reaktionsgemisch zur Bildung von Nebenprodukten neigt. Vorzugsweise beträgt die genannte mittlere Verweilzeit 1 bis 30 und insbesondere 2 bis 15 Minuten. Dies ist vor allem bei der Arbeitsweise mit rückgeführtem, gekühltem Reaktionsgemisch zu beachten.

Wie bereits oben erwähnt, soll die Temperatur bei der Trennung des flüssigen Reaktionsgemisches und des Gases 20 bis 60 °C betragen, die Trennung kann bei einem Druck von circa 10 bis circa 120 kPa erfolgen. Drucke unter 10 kPa sind prinzipiell möglich und für Abscheidung von 1,4-Dioxan auch günstig, jedoch bedingen sie im allgemeinen einen durch die zusätzliche Wirkung nicht mehr zu rechtfertigenden höheren apparativen Aufwand, Drucke über 120 kPa bringen keine Verbesserung, vorzugsweise wird bei normalen Atmosphärendruck gearbeitet.

Das vom flüssigen Reaktionsgemisch abgetrennte Gas enthält neben den zur Mischung mit $SO_3$ verwendeten Inertgasen Dämpfe des Zusatzstoffs beziehungsweise der Zusatzstoffe und des 1,4-Dioxans. Im allgemeinen lohnt eine Wiedergewinnung des 1,4-Dioxans wie auch der Zusatzstoffe nicht, diese werden zweckmäßig, beispielsweise mit Wasser, ausgewaschen und das Restgas in die Atmosphäre abgegeben, während das Waschwasser einer biologischen Klärung zugeführt wird. Falls n-Heptan verwendet wird, kann das Gas einer Verbrennung zugeführt werden.

Wie bereits oben aufgeführt, ermöglicht es das erfindungsgemäße Verfahren mit vergleichsweise geringem apparativen Aufwand und geringen zusätzlichen Kosten den 1,4-Dioxan-Gehalt, der mit $SO_3$ umgesetzten flüssigen Alkanol- beziehungsweise Alkylphenol-Oxethylate deutlich herabzusetzen, so daß die Umsetzungsprodukte nach der üblichen Neutralisation oft ohne weitere Nachbehandlung oder zumindest mit wesentlich herabgesetztem Nachbehandlungsaufwand verwendet werden können.

Nachfolgende Beispiele sollen die Erfindung näher erläutern:

Vergleichsversuch A

In einem Fallfilmreaktor mit anschließendem Quenchraum werden 1 036 Gew.-Teile pro Stunde eines Fettalkohol-Oxethylates, das im Mittel zwei -$CH_2CH_2O$-Gruppen enthält, eine OH-Zahl von 195, eine Molmasse von 288 und eine C-Kettenverteilung des Fettalkohols von $C_{10}$ = circa 1 %; $C_{12}$ = 54 ± 3 %, $C_{14}$ = 44 ± 3 % und $C_{16}$ = circa 1 % aufweist, mit 283 Gew.-Teilen pro Stunde $SO_3$ in Form einer 2,2 Vol.-% $SO_3$ enthaltenden Mischung mit Luft zur Reaktion gebracht. Auf 1 mol OH-Gruppen im Fettalkohol-Oxethylat werden 0,98 mol $SO_3$

eingesetzt. Das den Quenchraum verlassende Schwefelsäurehalbester/Luftgemisch wird in einen Zyklon gefördert und dort bei einer Temperatur von 35 °C unter normalem Atmosphärendruck in Gas und Flüssigkeit getrennt. Wenn sich im Zyklon circa 230 Gew.-Teile Flüssigkeit angesammelt haben, wird diese Flüssigkeit über einen Kühler zurück in den Quenchraum des Fallfilmreaktors gepumpt und dort durch schnelles Einströmenlassen intensiv mit dem aus dem Fallfilmreaktor zufließenden Reaktionsgemisch durchmischt. Aus dem Flüssigkeitskreislauf werden unter Konstanthaltung der 230 Gew.-Teile Inhalt des Hydrozyklons 1 324 Gew.-Teile pro Stunde, im wesentlichen Schwefelsäurehalbester enthaltende Flüssigkeit, die eine Säurezahl von 149,7 (theoretische Säurezahl 152,8) aufweist, abgezogen und der Neutralisation zugeführt. Die mittlere Verweilzeit der im wesentlichen Schwefelsäurehalbester enthaltenden Flüssigkeit zwischen der Beendigung der Reaktion mit $SO_3$ und der Neutralisation beträgt 15 Minuten. Bei der Neutralisation wird die im wesentlichen Schwefelsäurehalbester enthaltende Flüssigkeit mit 218 Gew.-Teilen pro Stunde einer wäßrigen Natronlauge, die 50 Gew.-% NaOH enthält, unter Zumischung von 417 Gew.-Teilen pro Stunde Wasser neutralisiert. Es wird so eine wasserhaltige Paste erhalten, die 70 Gew.-%, bezogen auf die Paste, Ethersulfat enthält. Von dieser Paste wird der Gehalt an 1,4-Dioxan zu 0,0076 Gew.-%, bezogen auf 100 % Ethersulfat, ermittelt. - Von der im wesentlichen Schwefelsäurehalbester enthaltenden gekühlten Flüssigkeit werden 35 000 Gew.-Teile pro Stunde zurückgeführt und in den Quenchraum nach dem Fallfilmreaktor eingeleitet, das sind 33,8 Gew.-Teile pro Stunde rückgeführte Flüssigkeit je 1 Gew.-Teil pro Stunde eingesetztes Fettalkohol-Oxethylat.

Beispiel 1

Es wird verfahren wie im Vergleichsversuch A angegeben, jedoch werden der zurückgeführten, im wesentlichen Schwefelsäurehalbester enthaltenden Flüssigkeit nach der Kühlung und nach der Abführung des Teils, der zur Neutralisation geht, 3 Gew.-Teile pro Stunde, das sind 0,29 Gew.-% Wasser, bezogen auf eingesetztes Fettalkohol-Oxethylat, zugesetzt und nachfolgend in einem statischen Mischer intensiv durchmischt. Die Temperatur bei der Trennung von Flüssigkeit und Gas beträgt wiederum 35 °C unter normalem Atmosphärendruck. Nach Einstellung des Gleichgewichtes werden in der im wesentlichen Schwefelsäurehalbester enthaltenden Flüssigkeit, die den Zyklon verläßt, 0,17 Gew.-% Wasser festgestellt. Um eine entsprechende Menge wird das bei der Neutralisation zugesetzte Wasser gekürzt. In der so erhaltenen 70 Gew.-% Ethersulfat enthaltenden Paste wird ein Gehalt an 1,4-Dioxan von 0,0034 Gew.-%, bezogen auf 100 % Ethersulfat, festgestellt.

Beispiel 2

Es wird verfahren wie in Beispiel 1, jedoch werden anstelle von 3 Gew.-Teile pro Stunde 12 Gew.-Teile pro Stunde Wasser, das sind 1,2 Gew.-%, bezogen auf eingesetztes Fettalkohol-Oxethylat, in den Kreislauf der gekühlten, im wesentlichen Schwefelsäurehalbester enthaltenden Flüssigkeit eingespeist. Der Gehalt der 70 gew.%igen wäßrigen Ethersulfatpaste an 1,4-Dioxan beträgt 0,0019 Gew.-%, bezogen auf 100 % Ethersulfat.

Vergleichsversuch B

In einem Fallfilmreaktor mit anschließendem Quenchraum werden 811 Gew.-Teile pro Stunde eines Fettalkohol-Oxethylates, das im Mittel 2 -$CH_2CH_2O$-Gruppen enthält, eine OH-Zahl von 201,8, eine Molmasse von 278 und eine C-Kettenverteilung des Fettalkohols von $C_{10}$ = circa 1,5 %; $C_{12}$ = 71 ± 3 %; $C_{14}$ = 26 ± 3 % und $C_{16}$ = 1,5 % aufweist, mit 226,4 Gew.-Teilen pro Stunde $SO_3$ in Form einer 1,8 Vol.-% $SO_3$ enthaltenden Mischung mit Luft zur Reaktion gebracht. Auf 1 mol OH-Gruppen im Fettalkohol-Oxethylat werden 0,97 mol $SO_3$ eingesetzt. Das den Quenchraum verlassende Schwefelsäurehalbester/Luftgemisch wird in einen Zyklon gefördert und dort bei einer Temperatur von 35 °C unter normalem Atmosphärendruck in Gas und Flüssigkeit getrennt. Wenn sich im Zyklon circa 10 Gew.-Teile Flüssigkeit angesammelt haben, wird diese Flüssigkeit über einen Kühler zurück in den Quenchraum des Fallfilmreaktors gepumpt und dort durch schnelles Einströmenlassen intensiv mit dem aus dem Fallfilmreaktor zufließenden Reaktionsgemisch durchmischt. Aus dem Flüssigkeitskreislauf werden unter Konstanthaltung der 10 Gew.-Teile Inhalt des Hydrozyklons 1 035 Gew.-Teile pro Stunde, im wesentlichen Schwefelsäurehalbester enthaltende Flüssigkeit, die eine Säurezahl von 152,0 aufweist, abgezogen und der Neutralisation zugeführt.

Die mittlere Verweilzeit der im wesentlichen Schwefelsäurehalbester enthaltenden Flüssigkeit zwischen der Beendigung der Reaktion mit $SO_3$ und der Neutralisation beträgt 5 Minuten. Bei der Neutralisation wird die im wesentlichen Schwefelsäurehalbester enthaltende Flüssigkeit mit 227 Gew.-Teilen pro Stunde einer wäßrigen Natronlauge, die 50 Gew.-% NaOH enthält, unter Zumischung von 2 680 Gew.-Teilen Wasser pro Stunde neutralisiert. Es wird eine wäßrige Lösung erhalten, die 28 Gew.-%, bezogen auf die wäßrige Lösung, Ethersulfat enthält. Von dieser wäßrigen Lösung wird der Gehalt an 1,4-Dioxan zu 0,0083 Gew.-%, bezogen auf 100 % Ethersulfat, ermittelt. - Von der im wesentlichen Schwefelsäurehalbester enthaltenden gekühlten Flüssigkeit werden 35 000 Gew.-Teile pro Stunde zurückgeführt und in den Quenchraum nach dem Fallfilmreaktor eingeleitet, das sind 43,1 Gew.-Teile pro Stunde rückgeführte Flüssigkeit je 1 Gew.-Teil pro Stunde eingesetztes Fettalkohol-Oxethylat.

Beispiel 3

Es wird verfahren wie in Vergleichsversuch B angegeben, jedoch werden der zurückgeführten, im wesentlichen Schwefelsäurehalbester enthaltenden Flüssigkeit nach der Kühlung und nach der Abführung des Teils, der zur Neutralisation geht, 15 Gew.-Teile pro Stunde Wasser, das sind 1,85 Gew.-%, bezogen auf eingesetztes Fettalkohol-Oxethylat, zugesetzt und nachfolgend in einem statischen Mischer intensiv durchmischt. Die Temperatur bei der Trennung von Flüssigkeit und Gas beträgt wiederum 35 °C unter normalem Atmosphärendruck. Nach Einstellung des Gleichgewichts werden in der im wesentlichen Schwefelsäurehalbester enthaltenden Flüssigkeit, die den Zyklon verläßt, 0,7 Gew.-% Wasser festgestellt. Um eine entsprechende Menge wird das bei der Neutralisation zugesetzte Wasser gekürzt. In der so erhaltenen, 28 Gew.-% Ethersulfat enthaltenden Lösung wird ein Gehalt an 1,4-Dioxan von 0,0017 Gew.-%, bezogen auf 100 % Ethersulfat, festgestellt.

Vergleichsversuch C

Es wird verfahren wie in Vergleichsversuch B, mit folgenden Unterschieden:
Es werden 955 Gew.-Teile pro Stunde eines Fettalkohol-Oxethylates, das im Mittel 3 -$CH_2CH_2O$-Gruppen enthält, eine OH-Zahl von 173, eine Molmasse von 324 und eine C-Kettenverteilung des Fettalkohols von $C_{10}$ = circa 1 %; $C_{12}$ = 54 ± 4 %; $C_{14}$ = 44 ± 3 % und $C_{16}$ = circa 1 %, mit 226,4 Gew.-Teilen pro Stunde $SO_3$ in Form einer 1,8 Vol.-% $SO_3$ enthaltenden Mischung mit Luft zur Reaktion gebracht. Auf 1 mol OH-Gruppen im Fettalkohol-Oxethylat werden 0,97 mol $SO_3$ eingesetzt. Zur Neutralisation des Schwefelsäurehalbesters werden 227 Gew.-Teile pro Stunde einer wäßrigen Natronlauge, die 50 Gew.-% NaOH enthält, und 373 Gew.-Teile pro Stunde Wasser verwendet. Die so erhaltene wasserhaltige Ethersulfatpaste hat einen Gehalt von 1,4-Dioxan von 0,0167 Gew.-%, bezogen auf 100 % Ethersulfat. - Wie im Vergleichsversuch B werden von der im wesentlichen Schwefelsäurehalbester enthaltenen gekühlten Flüssigkeit 35 000 Gew.-Teile pro Stunde zurückgeführt und in den Quenchraum nach dem Fallfilmreaktor eingeleitet, das sind 36,6 Gew.-Teile pro Stunde rückgeführte Flüssigkeit je 1 Gew.-Teil pro Stunde eingesetztes Fettalkohol-Oxethylat.

Beispiel 4

Es wird verfahren wie in Vergleichsversuch C angegeben, jedoch werden der zurückgeführten, im wesentlichen Schwefelsäurehalbester enthaltenden Flüssigkeit nach der Kühlung und nach der Abführung des Teils, der zur Neutralisation geht, 10 Gew.-Teile pro Stunde, das sind 1,05 Gew.-% Wasser, bezogen auf eingesetztes Fettalkohol-Oxethylat, zugesetzt und nachfolgend in einem statischen Mischer intensiv durchmischt. Die Temperatur bei der Trennung von Flüssigkeit und Gas beträgt 35 °C unter normalem Atmosphärendruck. Nach Einstellung des Gleichgewichtes werden in der im wesentlichen Schwefelsäurehalbester enthaltenden Flüssigkeit, die den Zyklon verläßt, 0,45 Gew.-% Wasser festgestellt. In der nach der Neutralisation erhaltenen wäßrigen Ethersulfatpaste wird ein Gehalt an 1,4-Dioxan von 0,0066 Gew.-%, bezogen auf 100 % Ethersulfat, ermittelt.

Vergleichsversuch D

Es wird verfahren wie in Vergleichsversuch C beschrieben, jedoch wird das $SO_3$ als 3,8 vol.%ige Mischung mit Luft eingesetzt. Das aus dem Reaktorsystem austretende, im wesentlichen Schwefelsäurehalbester enthaltende Flüssigkeitsgasgemisch wird in einem Zyklon getrennt und 70 000 Gew.-Teile pro Stunde, das sind 73,3 Gew.-Teile pro Stunde je 1 Gew.-Teil pro Stunde eingesetztes Alkohol-Oxethylat, der vom Gas befreiten, im wesentlichen Schwefelsäurehalbester enthaltenden Flüssigkeit über einen Kühler in den nach dem Reaktor angeordneten Quenchraum zurückgeführt. Die mittlere Verweilzeit der im wesentlichen Schwefelsäurehalbester enthaltenden Flüssigkeit zwischen der Beendigung der Reaktion mit $SO_3$ und der Neutralisation beträgt 5 Minuten. Die Temperatur bei der Trennung der Flüssigkeit von dem Gas liegt bei 30 °C. Es werden 1 190 Gew.-Teile pro Stunde der im wesentlichen Schwefelsäurehalbester enthaltenden Flüssigkeit abgeführt und mit 227 Gew.-Teilen pro Stunde einer wäßrigen, 50 Gew.-% NaOH enthaltenden Natronlauge und 373 Gew.-Teilen pro Stunde Wasser, neutralisiert. Der 1,4-Dioxangehalt der nach der Neutralisation erhaltenen Ethersulfatpaste beträgt 0,0123 Gew.-%, bezogen auf 100 % Ethersulfat.

Beispiel 5

Es wird verfahren wie in Vergleichsversuch D angegeben, jedoch werden der zurückgeführten, im wesentlichen Schwefelsäurehalbester enthaltenden Flüssigkeit nach der Kühlung und nach der Abführung des Teils, der zur Neutralisation geht, 10 Gew.-Teile pro Stunde, das sind 1,04 Gew.-% Wasser, bezogen auf eingesetztes Fettalkohol-Oxethylat, zugesetzt und nachfolgend in einem statischen Mischer intensiv durchmischt. Die Temperatur bei der Trennung von Flüssigkeit und Gas beträgt 30 °C unter normalem Atmosphärendruck. Nach Einstellung des Gleichgewichtes werden in der im wesentlichen Schwefelsäurehalbester enthaltenden Flüssigkeit, die den Zyklon verläßt, 0,9 Gew.-% Wasser festgestellt. Um eine entsprechende Menge wird das bei der Neutralisation zugesetzte Wasser gekürzt. In der so erhaltenen Ethersulfatpaste wird ein Gehalt an 1,4-Dioxan von 0,0023 Gew.-%, bezogen auf 100 % Ethersulfat, festgestellt.

Vergleichsversuch E

Es wird verfahren wie in Vergleichsversuch A, jedoch werden von der im wesentlichen Schwefelsäurehalbester enthaltenden gekühlten Flüssigkeit 20 000 Gew.-Teile pro Stunde zurückgeführt und in den Quenchraum nach dem Fallfilmreaktor eingeleitet, das sind 19,3 Gew.-Teile rückgeführte Flüssigkeit je 1 Gew.-Teil eingesetztes Fettalkohol-Oxethylat. Die Temperatur bei der Trennung von Flüssigkeit und Gas beträgt 30 °C unter normalem Atmosphärendruck. Die Neutralisation erfolgt wie in Vergleichsversuch A beschrieben. Es wird so eine wasserhaltige Paste erhalten, die 70 Gew.-%, bezogen auf die Paste, Ethersulfat enthält. Von dieser Paste wird der Gehalt an 1,4-Dioxan zu 0,0050 Gew.-%, bezogen auf 100 % Ethersulfat, ermittelt.

Beispiel 6

Es wird verfahren wie in Vergleichsversuch E angegeben, jedoch werden der zurückgeführten, im wesentlichen Schwefelsäurehalbester enthaltenden Flüssigkeit über eine Düse 19,5 Gew.-Teile pro Stunde, das sind 1,9 Gew.-% Wasser, bezogen auf eingesetztes Fettalkohol-Oxethylat, zugesetzt und nachfolgend in einem statischen Mischer intensiv durchmischt. Die Temperatur bei der Trennung von Flüssigkeit und Gas beträgt wiederum 30 °C unter normalem Atmosphärendruck. Nach Einstellung des Gleichgewichtes werden in der im wesentlichen Schwefelsäurehalbester enthaltenden Flüssigkeit, die den Zyklon verläßt, 0,7 Gew.-% Wasser festgestellt. Um eine entsprechende Menge wird das bei der Neutralisation zugesetzte Wasser gekürzt. In der so erhaltenen Ethersulfatpaste wird ein Gehalt an 1,4-Dioxan von 0,0008 Gew.-%, bezogen auf 100 % Ethersulfat, festgestellt.

**Patentansprüche**

1. Verfahren zur Herstellung sulfatierter Alkanol- oder Alkylphenol-Oxethylate, wobei mindestens ein flüssiges Alkanol-Oxethylat, das 6 bis 22 C-Atome im Alkanolrest enthält, oder mindestens ein Alkylphenol-Oxethylat, das 1 bis 3 Alkylgruppen und insgesamt 8 bis 18 C-Atome in den Alkylgruppen enthält, mit einem Gasgemisch, das neben mindestens einem Inertgas 1 bis 8 Vol.-%, bezogen auf das Gasgemisch, gasförmiges $SO_3$ enthält, im Verhältnis von 0,9 bis 1 mol $SO_3$ je 1 mol OH-Gruppen im Alkanol- oder Alkylphenol-Oxethylat unter Kühlung in Kontakt gebracht wird, nach Beendigung der Reaktion das flüssige Reaktionsgemisch und das Gas voneinander getrennt werden und das flüssige Reaktionsgemisch mit wäßrigem Alkalihydroxid, Magnesiumhydroxid, Ammoniumhydroxid oder substituiertem Ammoniumhydroxid neutralisiert wird, dadurch gekennzeichnet, daß dem Reaktionsgemisch nach Beendigung der Reaktion mit $SO_3$, jedoch vor der Trennung von flüssigem Reaktionsgemisch und Gas 0,1 bis 5 Gew.-%, bezogen auf eingesetztes Alkanol- oder Alkylphenol-Oxethylat, von mindestens einer der folgenden Verbindungen: Wasser, Ethanol, Propanol-1, Propanol-2, n-Heptan zugemischt werden, die mittlere Verweilzeit des Reaktionsgemisches von der Zugabe mindestens einer der genannten Verbindungen bis zur Abführung des flüssigen Reaktionsgemisches zur Neutralisation 60 Minuten nicht überschreitet und die Temperatur bei der Trennung von Flüssigkeit und Gas 20 bis 60 °C beträgt.

2. Verfahren nach Anspruch 1, wobei auf 1 Gew.-Teil pro Stunde eingesetztes Alkanol- oder Alkylphenol-Oxethylat 0,5 bis 100 Gew.-Teile pro Stunde des nach der Umsetzung mit $SO_3$ und Abtrennung des Gases erhaltenen flüssigen Reaktionsgemisches gekühlt, zurückgeführt und dem Reaktionsgemisch, bestehend aus Flüssigkeit und Gas, nach Beendigung der Reaktion mit $SO_3$ zugesetzt werden, dadurch gekennzeichnet, daß diesem zurückgeführten flüssigen Reaktionsgemisch mindestens eine der in Anspruch 1 genannten Verbindungen zugemischt wird.

3.  Verfahren nach Anspruch 1 oder 2, dadurch gekennzeichnet, daß als zugemischte Verbindung Wasser eingesetzt wird.

4.  Verfahren nach einem oder mehreren der Ansprüche 1 bis 3, dadurch gekennzeichnet, daß 0,2 bis 2 Gew.-%, bezogen auf eingesetztes Alkanol- oder Alkylphenol-Oxethylat, von mindestens einer der in Anspruch 1 genannten Verbindungen dem Reaktionsgemisch zugesetzt werden.

5.  Verfahren nach einem oder mehreren der Ansprüche 1 bis 4, dadurch gekennzeichnet, daß die Temperatur bei der Trennung von flüssigem Reaktionsgemisch und Gas 25 bis 45 °C beträgt.

6.  Verfahren nach einem oder mehreren der Ansprüche 1 bis 5, dadurch gekennzeichnet, daß mindestens ein Alkanol-Oxethylat eingesetzt wird, das im Alkanolteil 8 bis 18 C-Atome enthält.

7.  Verfahren nach einem oder mehreren der Ansprüche 1 bis 5, dadurch gekennzeichnet, daß mindestens ein Alkanol- oder Alkylphenol-Oxethylat verwendet wird, das 1 bis 6 -$CH_2CH_2O$-Gruppen enthält.

8.  Verfahren nach einem oder mehreren der Ansprüche 1 bis 7, dadurch gekennzeichnet, daß ein Gasgemisch verwendet wird, das 1,5 bis 5 Vol.-%, bezogen auf das Gasgemisch, gasförmiges $SO_3$ enthält.

9.  Verfahren nach einem oder mehreren der Ansprüche 1 bis 8, dadurch gekennzeichnet, daß die mittlere Verweilzeit von der Zugabe der in Anspruch 1 genannten Verbindungen zum Reaktionsgemisch bis zur Neutralisation 1 bis 30 Minuten beträgt.

10. Verfahren nach einem oder mehreren der Ansprüche 1 bis 9, dadurch gekennzeichnet, daß je ein Vol.-Teil des eingesetzten, $SO_3$ enthaltenden Gasgemisches 0,5 bis 3 Vol.-Teile von mindestens einem Inertgas dem Reaktionsgemisch nach Beendigung der Reaktion mit $SO_3$, jedoch vor der Trennung von flüssigem Reaktionsgemisch und Gas, zugegeben werden.

## Claims

1.  A process for the preparation of sulfated alkanol oxethylates or alkylphenol oxethylates, in which at least one liquid alkanol oxethylate having 6 to 22 C-atoms in its alkanol residue or at least one alkylphenol oxethylate having 1 to 3 alkyl groups and a total of 8 to 18 C-atoms in its alkyl groups is brought into contact, with cooling, with a gas mixture which contains in addition to at least one inert gas, 1 to 8 % by volume, relative to the gas mixture, of gaseous $SO_3$, in the ratio of 0.9 to 1 mol of $SO_3$ per mol of OH groups in the alkanol oxethylate or alkylphenol oxethylate, after completion of the reaction the liquid reaction mixture and the gas are separated from one another and the liquid reaction mixture is neutralized using aqueous alkali metal hydroxide, magnesium hydroxide, ammonium hydroxide or substituted ammonium hydroxide, characterized by admixing 0.1 to 5 % by weight, relative to the alkanol oxethylate or alkylphenol oxethylate employed, of at least one of the following compounds: water, ethanol, 1-propanol, 2-propanol or n-heptane to the reaction mixture after completion of the reaction with $SO_3$, but before the separation of liquid reaction mixture and gas, the mean residence time of the reaction mixture from the addition of at least one of the said compounds until the removal of the liquid reaction mixture for neutralization not exceeding 60 minutes and the temperature during the separation of liquid and gas being 20 to 60 °C.

2.  The process as claimed in claim 1, in which 0.5 to 100 parts by weight per hour of the liquid reaction mixture obtained after reaction with $SO_3$ and separation of the gas to 1 part by weight per hour of the alkanol oxethylate or alkylphenol oxethylate employed are cooled, recycled and added to the reaction mixture consisting of liquid and gas after completion of the reaction with $SO_3$, wherein at least one of the compounds mentioned in claim 1 is admixed to this recycled liquid reaction mixture.

3.  The process as claimed in claim 1 or 2, wherein water is employed as the admixed compound.

4.  The process as claimed in one or more of claims 1 to 3, wherein 0.2 to 2 % by weight relative to the alkanol oxethylate or alkylphenol oxethylate employed, of at least one of the compounds mentioned in claim 1 are added to the reaction mixture.

5. The process as claimed in one or more of claims 1 to 4, wherein the temperature in the separation of liquid reaction mixture and gas is 25 to 45°C.

6. The process as claimed in one or more of claims 1 to 5, wherein at least one alkanol oxethylate is employed which contains 8 to 18 carbon atoms in the alkanol moiety.

7. The process as claimed in one or more of claims 1 to 5, wherein at least one alkanol oxethylate or alkylphenol oxethylate which contains 1 to 6 -$CH_2CH_2O$- groups is used.

8. The process as claimed in one or more of claims 1 to 7, wherein a gas mixture is used which contains 1.5 to 5 % by volume, relative to the gas mixture, of gaseous $SO_3$.

9. The process as claimed in one or more of claims 1 to 8, wherein the mean residence time from the addition of the compounds mentioned in claim 1 to the reaction mixture until neutralization is 1 to 30 minutes.

10. The process as claimed in one or more of claims 1 to 9, wherein 0.5 to 3 parts by volume of at least one inert gas are added to the reaction mixture per part by volume of the $SO_3$-containing gas mixture employed, after completion of the reaction with $SO_3$, but before the separation of liquid reaction mixture and gas.


**Revendications**

1. Procédé pour préparer des oxyéthylats sulfatés d'alcanols ou d'alkyl-phénols selon lequel on met en contact, tout en refroidissant, au moins un oxyéthylat d'alcanol liquide contenant de 6 à 22 atomes de carbone dans le radical d'alcanol, ou au moins un oxyéthylat d'alkyl-phénol contenant de 1 à 3 radicaux alkyles et au total de 8 à 18 atomes de carbone dans les radicaux alkyles, avec un mélange gazeux qui, en plus d'au moins un gaz inerte, contient de 1 à 8% en volume, par rapport au mélange gazeux, de $SO_3$ gazeux, dans un rapport de 0,9 à 1 mol de $SO_3$ par mole de radicaux OH dans l'oxyéthylat d'alcanol ou d'alkyl-phénol, puis, la réaction terminée, on sépare l'un de l'autre Le mélange réactionnel liquide et le gaz et on neutralise le mélange réactionnel liquide avec un hydroxyde de métal alcalin, de l'hydroxyde de magnésium, de l'hydroxyde d'ammonium ou un hydroxyde d'ammonium substitué aqueux, procédé caractérisé en ce qu'on ajoute au mélange réactionnel, après la fin de la réaction avec $SO_3$ mais avant la séparation du mélange réactionnel liquide d'avec le gaz, de 0,1 à 5% en poids, par rapport à l'oxyéthylat d'alcanol ou d'alkyl-phénol mis en jeu, d'au moins un des composés suivants : eau, éthanol, propanol-1, propanol-2 et n-heptane, en ce que le temps de séjour moyen du mélange réactionnel, allant de l'addition d'au moins un des composés cités jusqu'à l'évacuation du mélange réactionnel liquide pour la neutralisation, ne dépasse pas 60 minutes et en ce que la température, lors de la séparation du liquide et du gaz, est comprise entre 20 et 60°C.

2. Procédé selon la revendication 1 selon lequel, pour 1 partie en poids par heure de l'oxyéthylat d'alcanol ou d'alkyl-phénol mis en jeu, on refroidit de 0,5 à 100 parties en poids par heure du mélange réactionnel liquide obtenu après la réaction avec $SO_3$ et après la séparation du gaz, on les recycle et on les ajoute au mélange réactionnel constitué du liquide et du gaz, après la fin de la réaction avec $SO_3$, ledit procédé étant caractérisé en ce qu'on ajoute au moins un des composés cités à la revendication 1 à ce mélange réactionnel liquide recyclé.

3. Procédé selon l'une des revendications 1 et 2 caractérisé en ce qu'on utilise l'eau comme composé ajouté.

4. Procédé selon une ou plusieurs des revendications 1 à 3, caractérisé en ce qu'on ajoute au mélange réactionnel de 0,2 à 2% en poids, par rapport à l'oxyéthylat d'alcanol ou d'alkyl-phénol mis en jeu, d'au moins un des composés cités à la revendication 1.

5. Procédé selon une ou plusieurs des revendications 1 à 4, caractérisé en ce que la température, lors de la séparation du mélange réactionnel liquide et du gaz, est comprise entre 25 et 45°C.

6. Procédé selon une ou plusieurs des revendications 1 à 5, caractérisé en ce qu'on utilise au moins un oxyéthylat d'alcanol dont la partie alcanol contient de 8 à 18 atomes de carbone.

7. Procédé selon une ou plusieurs des revendications 1 à 5, caractérisé en ce qu'on utilise au moins un oxyéthylat d'alcanol ou d'alkyl-phénol contenant de 1 à 6 radicaux -$CH_2CH_2O$-.

8. Procédé selon une ou plusieurs des revendications 1 à 7, caractérisé en ce qu'on utilise un mélange gazeux qui contient de 1,5 à 5% en volume de $SO_3$ gazeux, par rapport au mélange gazeux.

9. Procédé selon une ou plusieurs des revendications 1 à 8, caractérisé en ce que le temps de séjour moyen entre l'addition, au mélange réactionnel, des composés cités à la revendication 1 et la neutralisation est compris entre 1 et 30 minutes.

10. Procédé selon une ou plusieurs des revendications 1 à 9, caractérisé en ce que, par partie en volume du mélange gazeux contenant $SO_3$ mis en jeu, on ajoute au mélange réactionnel de 0,5 à 3 parties en volume d'au moins un gaz inerte, après la fin de la réaction avec $SO_3$ mais avant la séparation du mélange réactionnel liquide et du gaz.